Europäisches Patentamt

(19) European Patent Office

Office européen des brevets

(11) Publication number: **0 193 282**
**B1**

(12) **EUROPEAN PATENT SPECIFICATION**

(51) Int. Cl.⁵: **C01B 33/34,** B01J 29/28, C07C 2/66, C07C 6/12, C07C 5/27, C07C 1/20, C07C 1/207

(45) Date of publication of the patent specification:
05.12.90

(21) Application number: 86300581.5

(22) Date of filing: 29.01.86

(54) A crystalline silicate and method of its synthesis.

(30) Priority: 26.02.85 US 705821
27.06.85 US 749242
27.06.85 US 749240

(43) Date of publication of application:
03.09.86 Bulletin 86/36

(45) Publication of the grant of the patent:
05.12.90 Bulletin 90/49

(84) Designated Contracting States:
BE DE FR GB IT NL SE

(56) References cited:
**No relevant documents have been disclosed**

(73) Proprietor: **MOBIL OIL CORPORATION, 150 East 42nd Street, New York New York 10017(US)**

(72) Inventor: **Valyocsik, Ernest William, 960 Randolph Drive, Yardley Pennsylvania 19067(US)**
Inventor: **Olson, David Harold, 11 Morningside Drive, Pennington New Jersey 08534(US)**
Inventor: **Rodewald, Paul Gerhard, 4 Merritt Lane, Rocky Hill New Jersey 08553(US)**

(74) Representative: **Cooper, John Anthony et al, Mobil Court 3 Clements Inn, London WC2A 2EB(GB)**

ACTORUM AG

## Description

This invention relates to a crystalline silicate and method of its synthesis.

Zeolitic materials, both natural and synthetic, have been demonstrated in the past to have catalytic properties for various types of hydrocarbon conversion. Certain zeolitic materials are ordered, porous crystalline aluminosilicates having a definite crystalline structure as determined by X-ray diffraction, within which there are a large number of smaller cavities which may be interconnected by a number of still smaller channels or pores. These cavities and pores are uniform in size within a specific zeolitic material. Since the dimensions of these pores are such as to accept for adsorption molecules of certain dimensions while rejecting those of larger dimensions, these materials have come to be known as "molecular sieves" and are utilized in a variety of ways to take advantage of these properties.

Such molecular sieves, both natural and synthetic, include a wide variety of positive ion-containing crystalline aluminosilicates. These aluminosilicates can be described as a rigid three-dimensional framework of $SiO_4$ and $AlO_4$ in which the tetrahedra are cross-linked by the sharing of oxygen atoms whereby the ratio of the total aluminum and silicon atoms to oxygen atoms is 1:2. The electrovalence of the tetrahedra containing aluminum is balanced by the inclusion in the crystal of a cation, for example an alkali metal or an alkaline earth metal cation. This can be expressed wherein the ratio of aluminum to the number of various cations, such as Ca/2, Sr/2, Na, K or Li, is equal to unity. One type of cation may be exchanged either entirely or partially with another type of cation utilizing ion exchange techniques in a conventional manner. By means of such cation exchange, it has been possible to vary the properties of a given aluminosilicate by suitable selection of the cation.

Prior art techniques have resulted in the formation of a great variety of synthetic zeolites. The zeolites have come to be designated by letter or other convenient symbols, as illustrated by zeolite A (U. S. Patent 2,882,243), zeolite X (U.S. Patent 2,882,244), zeolite Y (U. S. Patent 3,l30,007), zeolite ZK-5 (U. S. Patent 3,247,195), zeolite ZK-4 (U. S. Patent 3,314,752), zeolite ZSM-5 (U. S. Patent 3,702,886), zeolite ZSM-11 (U. S. Patent 3,709,979), zeolite ZSM-12 (U. S. Patent 3,832,449), zeolite ZSM-20 (U. S. Patent 3,972,983), ZSM-35 (U. S. Patent 4,0l6,245), ZSM-38 (U. S. Patent 4,046,859), and zeolite ZSM-23 (U. S. Patent 4,076,842).

The $SiO_2/Al_2O_3$ ratio of a given zeolite is often variable. For example, zeolite X can be synthesized with $SiO_2/Al_2O_3$ ratios of from 2 to 3; zeolite Y, from 3 to about 6. In some zeolites, the upper limit of the $SiO_2/Al_2O_3$ ratio is unbounded. ZSM-5 is one such example wherein the $SiO_2/Al_2O_3$ ratio is at least 5 and up to infinity. U. S. Patent 3,94l,871 (Re. 29,948) discloses a porous crystalline silicate made from a reaction mixture containing no deliberately added alumina in the recipe and exhibiting the X-ray diffraction pattern characteristic of ZSM-5.

In a first aspect, the present invention resides in a novel porous crystalline material, designated as "ZSM-58", having an X-ray diffraction pattern exhibiting values substantially as set forth in Table 1 below.

In a second aspect, the invention resides in a method of preforming ZSM-58 comprising preparing a reaction mixture containing sources of an alkali metal or alkaline earth metal oxide, an oxide of silicon, an oxide of aluminum, water and methyltropinium salt directing agent of the formula (I), wherein X is an anion, and maintaining said reaction mixture under crystallization conditions until crystals of said material are formed.

In the as-synthesized form, ZSM-58 has a formula, on an anhydrous basis and in terms of moles of oxides per 100 moles of silica, as follows:

$(0.1-2.0)R_2O:(0.02-1.0)M_{2/n}O:(0.1-2)Al_2O_3:(100)SiO_2$ wherein M is an alkali or alkaline earth metal, n is the valence of M, and R is an organic cation of a methyltropinium salt. The typical X-ray diffraction pattern intensities for ZSM-58 are shown in Table 1 below.

## TABLE 1

| Interplanar d-Spacing (A) | | Relative Intensity, I/Io |
|---|---|---|
| 13.70 | $\pm$ 0.20 | W |
| 11.53 | $\pm$ 0.20 | W-VS |
| 10.38 | $\pm$ 0.20 | W |
| 7.82 | $\pm$ 0.14 | W-VS |
| 6.93-6.79 | $\pm$ 0.14 | W-VS |
| 6.19 | $\pm$ 0.14 | W-VS |
| 5.94 | $\pm$ 0.12 | W-M |
| 5.77 | $\pm$ 0.12 | VS |
| 5.22 | $\pm$ 0.12 | W |
| 5.18 | $\pm$ 0.10 | VS |
| 4.86 | $\pm$ 0.09 | M-S |
| 4.72 | $\pm$ 0.08 | S |
| 4.57 | $\pm$ 0.08 | W |
| 4.51 | $\pm$ 0.08 | S |
| 4.43 | $\pm$ 0.08 | W |
| 4.19 | $\pm$ 0.08 | W |
| 4.15 | $\pm$ 0.08 | M |
| 4.00 | $\pm$ 0.07 | W |
| 3.97 | $\pm$ 0.07 | W |
| 3.89 | $\pm$ 0.07 | W |
| 3.84 | $\pm$ 0.07 | M |
| 3.81 | $\pm$ 0.07 | W-M |
| 3.59 | $\pm$ 0.06 | W |
| 3.46 | $\pm$ 0.06 | W-M |
| 3.41 | $\pm$ 0.06 | S-VS |
| 3.36 | $\pm$ 0.06 | S-VS |
| 3.32 | $\pm$ 0.06 | M-S |
| 3.29 | $\pm$ 0.05 | W |
| 3.17 | $\pm$ 0.05 | W-M |
| 3.07 | $\pm$ 0.05 | W-M |
| 3.05 | $\pm$ 0.05 | W-M |
| 3.01 | $\pm$ 0.05 | W-M |
| 2.88 | $\pm$ 0.05 | W |
| 2.85 | $\pm$ 0.05 | W |
| 2.75 | $\pm$ 0.05 | W |
| 2.67 | $\pm$ 0.04 | W |
| 2.60 | $\pm$ 0.04 | W |

These values were determined by standard techniques. The radiation was the K-alpha doublet of copper and a diffractometer equipped with a scintillation counter and an associated computer was used. The peak heights, I, and the positions as a function of 2 theta, where theta is the Bragg angle, were determined using algorithms on the computer associated with the spectrometer. From these, the relative intensities, 100 $I/I_o$, where $I_o$ is the intensity of the strongest line or peak, and d (obs.) the interplanar spacing in Angstrom Units (A), corresponding to the recorded lines, were determined. In Table 1, the relative intensities are given in terms of the symbols W=weak, M=medium, S=strong and VS=very strong. In terms of intensities, these may be generally designated as follows:

W = 0 - 20
M = 20 - 40
S = 40 - 60
VS = 60 - 100

It should be understood that this X-ray diffraction pattern is characteristic of all the species of ZSM-58 compositions. The sodium form as well as other cationic forms reveal substantially the same pattern with some minor shifts in interplanar spacing and variation in relative intensity. Other minor variations can occur, depending on the silicon to aluminum ratio of the particular sample, as well as its degree of thermal treatment. Multiplets may be observed in the typical X-ray pattern for ZSM-58 at d-spacing values of $6.93$-$6.79 \pm 0.14$, $4.86 \pm 0.09$, $3.41 \pm 0.06$, $3.07 \pm 0.05$ and $3.01 \pm 0.05$ Angstroms.

ZSM-58 is thermally stable and exhibits molecular shape selective properties as indicated by sorption tests.

The crystalline silicate ZSM-58 can be prepared from a reaction mixture containing sources of an alkali or alkaline earth metal oxide, an oxide of aluminum, an oxide of silicon, an organic cation of a methyltropinium salt, e.g. halide, hydroxide, sulfate, etc., and water, said reaction mixture having a composition, in terms of mole ratios of oxides, within the following ranges:

| Reactants | Useful | Preferred |
|---|---|---|
| $SiO_2/Al_2O_3$ | 50–1000 | 70–500 |
| $H_2O/SiO_2$ | 5–200 | 10–100 |
| $OH^-/SiO_2$ | 0–2.0 | 0.10–1.0 |
| $M/SiO_2$ | 0.01–3.0 | 0.10–1.0 |
| $R/SiO_2$ | 0.01–2.0 | 0.10–0.50 |

wherein R and M are as above defined.

Crystallization of ZSM-58 can be carried out under either static or stirred conditions in a suitable reactor vessel, such as for example, polypropylene jars or teflon (Registered trade mark) lined or stainless steel autoclaves. The total useful range of temperatures for crystallization is from 80°C to 225°C for a time sufficient for crystallization to occur at the temperature used, e.g. from 24 hours to 60 days. Thereafter, the crystals are separated from the liquid and recovered. The reaction mixture can be prepared utilizing materials which supply the appropriate oxides. Such materials may include sodium silicate, silica hydrosol, silica gel, silicic acid, sodium hydroxide, a source of aluminum, and the methyltropinium salt directing agent. The methyltropinium salt may be synthesized by selective methylation of 3-tropanol at the bridgehead nitrogen. This salt has the following formula (I), wherein X is an anion, such as, for example, a halide (e.g. iodide, chloride or bromide), nitrate, hydroxide, sulfate, bisulfate and perchlorate.

It should be realized that the reaction mixture oxides can be supplied by more than one source. The reaction mixture can be prepared either batchwise or continuously. Crystal size and crystallization time of the new crystalline material will vary with the nature of the reaction mixture employed and the crystallization conditions.

In all cases, synthesis of the ZSM-58 crystals is facilitated by the presence of at least 0.01 percent, preferably 0.10 percent and still more preferably 1 percent, seed crystals (based on total weight) of crystalline product.

The crystals prepared by the instant method can be shaped into a wide variety of particle sizes. Generally speaking, the particles can be in the form of a powder, a granule, or a molded product, such as an extrudate having particle size sufficient to pass through a 2 mesh (Tyler) screen and be retained on a 400 mesh (Tyler) screen. In cases where the catalyst is molded, such as by extrusion, the crystals can be extruded before drying or partially dried and then extruded.

The original alkali or alkaline earth metal cations of the as synthesized ZSM-58 can be replaced in accordance with techniques well known in the art, at least in part, by ion exchange with other cations. Preferred replacing cations include metal ions, hydrogen ions, hydrogen precursor, e.g. ammonium, ions and mixtures thereof. Particularly preferred cations are those which render the ZSM-58 catalytically active, especially for certain hydrocarbon conversion reactions. These include hydrogen, rare earth metals and metals of Groups IIA, IIIA, IVA, IB, IIB, IIIB, IVB and VIII of the Periodic Table of the Elements.

Typical ion exchange technique would be to contact the synthetic ZSM-58 with a salt of the desired replacing cation or cations. Examples of such salts include the halides, e.g. chlorides, nitrates and sulfates.

The crystalline silicate of the present invention can be used either in the alkali or alkaline earth metal form, e.g. the sodium or potassium form; the ammonium form; the hydrogen form or another univalent or multivalent cationic form. When used as a catalyst, ZSM-58 will be subjected to thermal treatment to remove part or all of any organic constituent.

The crystalline silicate can also be used as a catalyst in intimate combination with a hydrogenating component such as tungsten, vanadium, molybdenum, rhenium, nickel, cobalt, chromium, manganese, or a noble metal such as platinum or palladium where a hydrogenation-dehydrogenation function is to be performed. Such component can be exchanged into the composition to the extent aluminum is in the structure, impregnated therein or intimately physically admixed therewith. Such component can be impregnated in or on to it such as for example, by, in the case of platinum, treating the ZSM-58 with a solution containing a platinum metal-containing ion. Thus, suitable platinum compounds include chloroplatinic acid, platinous chloride and various compounds containing the platinum amine complex.

The above crystalline silicate, especially in its metal, hydrogen and ammonium forms can be beneficially converted to another form by thermal treatment. This thermal treatment is generally performed by heating one of these forms at a temperature of at least 370°C for at least 1 minute and generally not longer than 20 hours. While subatmospheric pressure can be employed for the thermal treatment, atmospheric pressure is desired for reasons of convenience. The thermal treatment can be performed at a temperature up to about 925°C. The thermally treated product is particularly useful in the catalysis of certain hydrocarbon conversion reactions.

The new silicate, when employed either as an adsorbent or as a catalyst in an organic compound conversion process should be dehydrated, at least partially. This can be done by heating to a temperature in the range of 200°C to 595°C in an inert atmosphere, such as air, nitrogen, etc. and at atmospheric, subatmospheric or superatmospheric pressures for between 30 minutes and 48 hours. Dehydration can also be performed at room temperature merely by placing ZSM-58 in a vacuum, but a longer time is required to obtain a sufficient amount of dehydration.

In practice, ZSM-58 can be used as a catalyst in the conversion of aromatic compounds selected from benzene and monocyclic alkyl-substituted benzene of from 7 to 12 carbon atoms having the formula (II), wherein R'' is methyl, ethyl or a combination thereof, and n is an integer of from 1 to 4. In other words, the feedstock aromatic compounds may be benzene, benzene containing from 1 to 4 methyl and/or ethyl group substituents, and mixtures thereof. Non-limiting examples of such feedstock compounds include benzene, toluene, xylene, ethylbenzene, mesitylene (1,3,5-trimethylbenzene), durene (1,2,4,5-tetramethylbenzene), pseudocumene (1,2,4-trimethylbenzene) and mixtures thereof.

Other reactant species may be present, such as for alkylation. Alkylating agent species include olefins such as ethylene, propylene, dodecylene, as well as formaldehyde, alkyl halides, alcohols and ethers; the alkyl portion thereof having from 1 to 24 carbon atoms. Numerous other acyclic compounds having at least one reactive alkyl radical may be utilized as alkylating agents.

Products of such aromatic conversion processes include alkyl-substituted benzene compounds which differ from the feedstock compounds depending upon the conversion effected. The following listing presents non-limiting examples:

| Feedstock Aromatic Compounds Include | Other Reactants Include | Product Aromatic Compounds Include |
|---|---|---|
| Benzene | Ethylene | Ethylbenzene |
| Toluene | Methanol | Xylene isomers |
| Xylene isomers, e.g., 9:73:18 wt. ratio of para:meta:ortho | --- | Different combination of xylene isomers, e.g. 23:57:20 wt. ratio of para:meta:ortho |
| Toluene | --- | Benzene and xylenes |
| Benzene | Propylene | Cumene and diisopropylbenzene |
| Toluene | Propylene | Cymene isomers |

Mechanisms of the present process may be isomerization alkylation, transalkylation and disproportionation. Disproportionation is a special case of transalkylation in which the alkylatable aromatic compound and the transalkylating agent is the same compound, for example, when toluene serves as the donor and acceptor of a transferred methyl group to produce benzene and xylene. Use of the term transalkylation includes the special case of disproportionation.

5

In general, the present process is conducted at conversion conditions including a temperature of from 150°C to 760°C, a pressure of from 100 to 20450 kPa (0 psig to 2950 psig), a weight hourly space velocity of from 0.1 to 2000 hr$^{-1}$ and a hydrogen/feedstock hydrocarbon compound mole ratio of from 0 (no added hydrogen) to 100.

Such aromatics conversion processes include isomerizing xylene feedstock components to a product enriched in p-xylene with reaction conditions including a temperature from 150 to 600°C, a pressure of from 100 to 7000 kPa (0 to 1000 psig), a weight hourly space velocity of from 0.1 to 200 hr$^{-1}$ and a hydrogen/hydrocarbon mole ratio of from 0 to 100; disproportionating toluene to a product comprising benzene and xylenes with reaction conditions including a temperature of from 200 to 760°C, a pressure of from 197 to 6310 kPa (14 to 900 psig) and a weight hourly space velocity of from 0.1 to 20 hr.$^{-1}$; alkylating aromatic hydrocarbons, e.g. benzene and alkylbenzenes, in the presence of an alkylating agent, e.g. olefins, formaldehyde, alkyl halides and alcohols, with reaction conditions including a temperature of from 150 to 650°C, a pressure of from 197 to 20450 kPa (14 to 2950 psig), a weight hourly space velocity of from 2 to 2000 hr$^{-1}$ and a feedstock aromatic hydrocarbon/alkylating agent mole ratio of from 1/1 to 20/1; and transalkylating aromatic hydrocarbons in the presence of polyalkylaromatic hydrocarbons with reaction conditions including a temperature of from 200 to 760°C, a pressure of from 197 to 20450 kPa (14 psig to 2950 psig), a weight hourly space velocity of from 10 to 1000 hr.$^{-1}$ and a feedstock aromatic hydrocarbon/polyalkylaromatic hydrocarbon mole ratio of from 1/1 to 16/1.

In addition, ZSM-58 can be used as a catalyst in the conversion of organic oxygenates selected from alcohols, carbonyls, ethers and mixtures thereof to hydrocarbons. Feedstock alcohols will be aliphatic alcohols of from 1 to 6 carbon atoms, preferably from 1 to 3 carbon atoms, e.g., methanol and ethanol. Feedstock carbonyls will be lower aliphatic carbonyls, such as, for example, acetone. Feedstock ethers will be lower aliphatic ethers of up to 6 carbon atoms, e.g., from 2 to 6 carbon atoms, such as dimethylether, n-propyl ether, p-dioxane, trioxane and hexose.

The product of such oxygenate conversion will be predominantly hydrocarbons including olefins of from 2 to 5 or more carbon atoms with $C_2$ olefins usually less than about 10% of the total and $C_{5+}$ olefins usually less than about 15% of the total. Aromatic hydrocarbons, such as durene, are also produced. $C_2$, $C_3$ and $C_4$ olefins are desired chemical products, and $C_{5+}$ products are valuable as gasoline components. In general, the reaction conditions employed will be a temperature of from 150° to 600°C, a pressure of from 50 to 5065 kPa (0.5 to 50 atmospheres) and a weight hourly space velocity of from 0.5 to 100/hr.$^{-1}$.

In the case of many catalytic uses, it is desirable to incorporate the ZSM-58 with another material resistant to the temperatures and other conditions employed in organic conversion processes. Such materials include active and inactive materials and synthetic or naturally occurring zeolites as well as inorganic materials such as clays, silica and/or metal oxides such as alumina. The latter may be either naturally occurring or in the form of gelatinous precipitates or gels including mixtures of silica and metal oxides. Use of a material in conjunction with the ZSM-58 crystal, i.e. combined therewith, which is active, tends to change the conversion and/or selectivity of the catalyst in certain organic conversion processes. Inactive materials suitably serve as diluents to control the amount of conversion in a given process so that products can be obtained economically and orderly without employing other means for controlling the rate of reaction. These materials may be incorporated into naturally occurring clays, e.g. bentonite and kaolin, to improve the crush strength of the catalyst under commercial operating conditions. Said materials, i.e. clays, oxides, etc., function as binders for the catalyst. It is desirable to provide a catalyst having good crush strength because in commercial use it is desirable to prevent the catalyst from breaking down into powder-like materials. These clay binders have been employed normally only for the purpose of improving the crush strength of the catalyst.

Naturally occurring clays which can be composited with the new crystal include the montmorillonite and kaolin family, which families include the subbentonites, and the kaolins commonly known as Dixie, McNamee, Georgia and Florida clays or others in which the main mineral constituent is halloysite, kaolinite, dickite, nacrite, or anauxite. Such clays can be used in the raw state as originally mined or initially subjected to calcination, acid treatment or chemical modification. Binders useful for compositing with the present crystal also include inorganic oxides, notably alumina.

In addition to the foregoing materials, the ZSM-58 crystal can be composited with a porous matrix material such as silica-alumina, silica-magnesia, silica-zirconia, silica-thoria, silica- beryllia, silica-titania as well as ternary compositions such as silica-alumina-thoria, silica-alumina-zirconia silica-alumina-magnesia and silica-magnesia-zirconia.

The relative proportions of finely divided crystalline material and inorganic oxide matrix vary widely, with the crystal content ranging from 1 to 90 percent by weight and more usually, particularly when the composite is prepared in the form of beads, in the range of 2 to 80 weight percent of the composite.

In order to more fully illustrate the nature of the invention and the manner of practicing same, the following examples are presented. In the examples, whenever sorption data are set forth for comparison of sorptive capacities for cyclohexane and/or n-hexane, they were determined as follows:

A weighed sample of the calcined ZSM-58 adsorbant was contacted with the desired pure adsorbate vapor in an adsorption chamber, evacuated to less than 1 mm and contacted with 80 mm Hg of n-hexane or cyclohexane vapor, pressures less than the vapor-liquid equilibrium pressure of the respective adsorbate at 90°C. The pressure was kept constant (within about ± 0.5 mm) by addition of adsorbate vapor controlled by a manostat during the adsorption period, which did not exceed about 8 hours. As adsorbate was adsorbed by the ZSM-58, the decrease in pressure caused the manostat to open a valve which admitted more adsorbate vapor to the chamber to restore the above control pressures. Sorption was complete when the pressure change was not sufficient to activate the manostat. The increase in weight was calculated as the adsorption capacity of the sample in g/100 g of calcined adsorbant.

When Alpha Value is examined, it is noted that the Alpha Value is an approximate indication of the catalytic cracking activity of the catalyst compared to a standard catalyst and it gives the relative rate constant (rate of normal hexane conversion per volume of catalyst per unit time). It is based on the activity of the highly active silica-alumina cracking catalyst taken as an Alpha of 1 (Rate Constant = 0.016 sec$^{-1}$). The Alpha Test is described in U.S. Patent 3,354,078 and in The Journal of Catalysis, Vol. IV, pp. 522-529 (August 1965). It is noted that intrinsic rate constants for many acid-catalyzed reactions are proportional to the Alpha Value for a particular crystalline silicate catalyst, i.e. the rates for toluene disproportionation, xylene isomerization, alkene conversion and methanol conversion (see "The Active Site of Acidic Aluminosilicate Catalysts," Nature, Vol. 309, No. 5969, pp. 589-591, 14 June 1984).

EXAMPLES 1 - 6

Six separate synthesis reaction mixtures were prepared with compositions indicated in Table 2. The mixtures were prepared with silica sol (30 percent SiO$_2$), NaAlO$_2$, NaOH, a methyltropinium salt, i.e. iodide, and water. The mixtures were maintained at 160°C for 4 days in a stainless steel, stirred (400 rpm) autoclave at autogenous pressure. Solids were separated from any unreacted components by filtration and then water washed, followed by drying at 110°C. The product crystals were analyzed by X-ray diffraction and chemical analysis. The product of Example 1 was found to be crystalline ZSM-58 with a trace of unidentified second component impurity. The products from Examples 2-6 proved to be 100 percent crystalline ZSM-58.

The X-ray diffraction pattern of the Example 4 crystals, after calcination at 538°C for 17 hours in air, is set forth as illustration in Table 3. Other properties of each crystalline product are presented in Table 4. In the latter table, compositions are calculated on the basis of 100 (SiO$_2$ + AlO$^-_4$) tetrahedra. The as-synthesized ZSM-58 from these examples contains from 3.8 to 5.0 methyltropinium cations per 100 tetrahedra.

## TABLE 2

| Example | Mixture Composition (mole ratios) | | | | |
| | $\dfrac{SiO_2}{Al_2O_3}$ | $\dfrac{H_2O}{SiO_2}$ | $\dfrac{OH^-}{SiO_2}$ | $\dfrac{Na^+}{SiO_2}$ | $\dfrac{R^*}{SiO_2}$ |
|---|---|---|---|---|---|
| 1 | 300 | 40 | 0.30 | 0.31 | 0.25 |
| 2 | 200 | 40 | 0.30 | 0.31 | 0.25 |
| 3 | 90 | 40 | 0.40 | 0.42 | 0.25 |
| 4 | 90 | 40 | 0.30 | 0.32 | 0.25 |
| 5 | 90 | 40 | 0.30 | 0.32 | 0.25 |
| 6 | 70 | 40 | 0.30 | 0.33 | 0.25 |

*R = methyltropinium cation.

## TABLE 3

| d(A) | Observed 2 Theta | Relative Intensity |
|---|---|---|
| 13.57425 | 6.511 | 7.4 |
| 11.44933 | 7.721 | 51.2 |
| 10.29541 | 8.588 | 4.1 |
| 7.76959 | 11.389 | 53.6 |
| 6.89736 | 12.834 | 60.1 |
| 6.84556 | 12.932 | 33.0 |
| 6.15999 | 14.378 | 57.8 |
| 5.91115 | 14.987 | 19.5 |
| 5.74071 | 15.435 | 85.8 |
| 5.16339 | 17.173 | 100.0 |
| 4.84326 | 18.317 | 51.9 |
| 4.70389 | 18.865 | 56.0 |
| 4.52632 | 19.612 | 20.3 |
| 4.49392 | 19.755 | 51.7 |
| 4.41905 | 20.093 | 4.7 |
| 4.13559 | 21.486 | 26.0 |
| 3.98517 | 22.307 | 11.8 |
| 3.96826 | 22.404 | 8.9 |
| 3.87191 | 22.969 | 17.1 |
| 3.82281 | 23.268 | 30.6 |
| 3.80712 | 23.365 | 25.6 |
| 3.57841 | 24.882 | 16.2 |
| 3.44668 | 25.849 | 35.2 |
| 3.38811 | 26.303 | 96.5 |
| 3.35769 | 26.546 | 86.7 |
| 3.34862 | 26.619 | 80.8 |
| 3.30859 | 26.947 | 66.2 |
| 3.28346 | 27.158 | 9.1 |
| 3.16039 | 28.237 | 23.3 |
| 3.06246 | 29.159 | 26.8 |
| 3.06070 | 29.176 | 31.2 |
| 3.03737 | 29.406 | 22.7 |
| 2.99654 | 29.816 | 25.2 |
| 2.98814 | 29.901 | 21.2 |
| 2.87045 | 31.158 | 4.1 |
| 2.84237 | 31.473 | 5.1 |
| 2.66429 | 33.638 | 5.5 |
| 2.58922 | 34.643 | 4.8 |
| 2.50349 | 35.869 | 4.3 |
| 2.48809 | 36.099 | 6.3 |
| 2.43821 | 36.863 | 9.0 |
| 2.42105 | 37.134 | 14.9 |
| 2.39052 | 37.626 | 5.8 |
| 2.35412 | 38.230 | 2.8 |

## TABLE 3
### (Continued)

| d(A) | Observed 2 Theta | Relative Intensity |
|---|---|---|
| 2.33296 | 38.591 | 4.3 |
| 2.30029 | 39.161 | 16.7 |
| 2.23686 | 40.319 | 2.4 |
| 2.23188 | 40.413 | 1.9 |
| 2.21126 | 40.807 | 3.2 |
| 2.16400 | 41.739 | 1.7 |
| 2.11106 | 42.836 | 1.4 |
| 2.07314 | 43.660 | 3.0 |
| 2.03910 | 44.427 | 0.3 |
| 1.97783 | 45.880 | 11.4 |
| 1.95022 | 46.568 | 4.4 |
| 1.93214 | 47.030 | 3.9 |
| 1.91503 | 47.476 | 3.7 |
| 1.83810 | 49.594 | 6.4 |
| 1.83554 | 49.667 | 5.5 |

## TABLE 4

| Example | Moles C / Mole N | Moles per Mole $Al_2O_3$ | | | COMPOSITION | | | |
|---|---|---|---|---|---|---|---|---|
| | | $N_2O$ | $Na_2O$ | $SiO_2$ | Al / $100T_d$ | $Na^+$ / $100T_d$ | $N^+$ / $100T_d$ | R / $100T_d$ |
| 1 | 9.5 | 4.09 | 0.85 | 223 | 0.89 | 0.76 | 3.6 | 3.8 |
| 2 | 11.2 | 2.43 | 0.74 | 140 | 1.4 | 1.0 | 3.4 | 4.2 |
| 3 | 9.6 | 1.85 | 0.13 | 83 | 2.4 | 0.30 | 4.4 | 4.7 |
| 4 | 10.2 | 1.69 | 0.12 | 78 | 2.5 | 0.30 | 4.2 | 4.8 |
| 5 | 10.8 | 1.77 | 0.25 | 85 | 2.3 | 0.58 | 4.1 | 4.9 |
| 6 | 9.6 | 1.50 | 0.10 | 62 | 3.1 | 0.30 | 4.7 | 5.0 |

EXAMPLE 7

A sample of the Example 4 product crystals, having been calcined in nitrogen for 4 hours at 500°C, ammonium exchanged and then converted to the hydrogen form, was subjected to the sorption test. Significant n-hexane, i.e. 8 weight percent at 90°C, was sorbed while only minimal cyclohexane (about 1 weight percent at 90°C) sorbed at 80 torr hexane partial pressure. This indicates molecular shape selectivity for the ZSM-58 of this invention.

EXAMPLE 8

The sample of Example 4 product used for sorption evaluation was evaluated in the Alpha Test. Its Alpha Value proved to be 13 at 538°C.

EXAMPLES 9-19

A feedstock comprising $C_8$ aromatic compounds, see Table 5, was passed over catalyst comprising the ZSM-58 product crystals from Example 4, calcined and converted to the hydrogen form in Example 7, at 483°C, 400 psig, (2859 kPa) a hydrogen/hydrocarbon ratio of 4/1 and a weight hourly space velocity varied for Examples 9-19 between 2 and 16. Reaction product compositions are given in Table 5. As can be seen from the data provided in Table 5, at a weight hourly space velocity of 4 hr⁻¹, near equilibrium (95%) p-xylene levels were achieved along with about 25% conversion of the ethylbenzene. Xylene loss levels were good, below about 3% at this conversion level.

## TABLE 5

| Example | Feed | 9 | 10 | 11 |
|---|---|---|---|---|
| Time on Steam(hr) | | 2.03 | 4.03 | 5.72 |
| Temp. (°C) | | 483 | 483 | 483 |
| WHSV (hr$^{-1}$) | | 4 | 4 | 8 |
| Liquid Product Analysis (wt. %) | | | | |
|    Light gas | | 0.74 | 0.60 | 0.62 |
|    Benzene | 0.4 | 4.43 | 4.05 | 2.88 |
|    Toluene | 0.10 | 1.44 | 1.42 | 0.79 |
|    Ethylbenzene (EB) | 24.74 | 18.94 | 18.57 | 20.64 |
|    p-xylene | 0.93 | 16.39 | 16.39 | 15.68 |
| | (1.2) | (22.6) | (22.7) | (21.5) |
|    m-xylene | 47.87 | 38.41 | 38.22 | 38.22 |
| | (63.7) | (53.0) | (53.0) | (52.4) |
|    o-xylene | 26.32 | 17.63 | 17.54 | 19.04 |
| | (35.0) | (24.3) | (24.3) | (26.1) |
| C$_9$+ (total) | | 1.99 | 3.06 | 2.10 |
| Total xylenes | 75.12 | 72.43 | 72.15 | 72.94 |
| p-xylene (% of Equilibrium.) | 5.2 | 94.3 | 94.6 | 89.6 |
| EB in Feed/EB converted (%) | | 23.4 | 24.9 | 16.6 |

TABLE 5 (continued)

| Example | 12 | 13 | 14 | 15 |
|---|---|---|---|---|
| Time on Steam(hr) | 9.72 | 14.74 | 16.26 | 17.78 |
| Temp. (°C) | 483 | 483 | 483 | 483 |
| WHSV ($hr^{-1}$) | 4 | 2 | 8 | 16 |
| Liquid Product Analysis (wt. %) | | | | |
| Light gas | 1.19 | 3.01 | 0.50 | 0.30 |
| Benzene | 4.88 | 7.56 | 2.30 | 1.27 |
| Toluene | 1.36 | 2.26 | 0.65 | 0.44 |
| Ethylbenzene (EB) | 18.19 | 16.66 | 21.36 | 22.77 |
| p-xylene | 16.44 | 15.94 | 14.19 | 10.35 |
| | (22.8) | (23.4) | (19.3) | (14.0) |
| m-xylene | 36.83 | 35.52 | 39.25 | 41.82 |
| | (51.1) | (52.1) | (53.3) | (56.4) |
| o-xylene | 18.86 | 16.75 | 20.14 | 22.04 |
| | (26.2) | (24.6) | (27.4) | (29.7) |
| $C_9$+ (total)2.10 | 2.23 | 2.29 | 1.57 | 0.98 |
| Total xylenes | 72.13 | 68.21 | 73.58 | 74.21 |
| p-xylene (% of Equilibrium.) | 95.0 | 97.37 | 80.4 | 58.1 |
| EB in Feed/EB converted (%) . | 26.5 | 32.7 | 13.7 | 8.0 |

## TABLE 5 (continued)

| Example | 16 | 17 | 18 | 19 |
|---|---|---|---|---|
| Time on Steam(hr) | 20.80 | 25.82 | 27.33 | 28.88 |
| Temp. (°C) | 483 | 483 | 483 | 483 |
| WHSV ($hr^{-1}$) | 4 | 2 | 8 | 16 |
| Liquid Product Analysis (wt. %) | | | | |
| Light gas | 0.47 | 1.58 | 0.37 | 0.26 |
| Benzene | 2.69 | 4.68 | 1.79 | 1.02 |
| Toluene | 0.91 | 1.50 | 0.54 | 0.39 |
| Ethylbenzene (EB) | 20.20 | 18.31 | 21.86 | 23.04 |
| p-xylene | 14.67 (20.0) | 15.44 (21.8) | 12.34 (16.7) | 8.29 (11.2) |
| m-xylene | 39.48 (53.8) | 37.04 (52.3) | 40.53 (54.8) | 43.32 (58.3) |
| o-xylene | 19.30 (26.3) | 18.34 (25.9) | 21.14 (28.6) | 22.74 (30.6) |
| C9+ (total) | 2.25 | 2.69 | 1.40 | 0.90 |
| Total xylenes | 73.45 | 70.82 | 74.01 | 74.35 |
| p-xylene (% of Equilibrium.) | 83.2 | 90.8 | 69.5 | 46.5 |
| EB in Feed/EB converted (%) | 18.4 | 26.0 | 11.6 | 6.9 |

EXAMPLES 20-21

Toluene was disproportionated over catalyst comprising the hydrogen-form of ZSM-58 prepared in Example 7 at two different weight hourly space velocities of 6 hr⁻¹ (Example 20) and 1 hr⁻¹ (Example 21). Reaction conditions included a temperature of 550°C and atmospheric pressure. Results, listed in Table 6, indicate that the ZSM-58 becomes more p-selective at lower toluene conversion.

12

## TABLE 6

| | | Selectivity, Wt. % | | | |
|---|---|---|---|---|---|
| Example | Conversion wt% | Benzene | p-xylene | m-xylene | o-xylene |
| 20 | 1.4 | 45 | 21[a] | 26 | 8 |
| 21 | 7.4 | 44 | 14[b] | 29 | 13 |

(a) p-xylene in xylenes=38% (Thermodynamic p-xylene in xylenes=25%)

(b) p-xylene in xylenes=25%

EXAMPLE 22

Alkylation of toluene with methanol alkylating agent was conducted over catalyst comprising the hydrogen-form of ZSM-58 prepared as in Example 7 at 500°C and atmospheric pressure. The weight hourly space velocity was maintained at 12 hr$^{-1}$ and the mole ratio of toluene to methanol was 4/1. Toluene conversion was 10 wt. % with significant selectivity to xylenes, especially the para-isomer, indicated. The xylenes product was composed of 58 wt % p-xylene, 25 wt % m-xylene and 17 wt % o-xylene. The 58 wt % para-isomer compares to a thermodynamic equilibrium value of only 25 wt %.

EXAMPLE 23

A feedstock comprising methanol was passed over 1.0 gram of hydrogen-form ZSM-58 product of Example 7 at conversion conditions including atmospheric pressure, 371°C and 4 hr$^{-1}$ WHSV. Conversion of the methanol was 100% with reaction product components listed below:

| Component | Wt. % |
|---|---|
| Methane | 2.6 |
| Ethane | 1.5 |
| Ethylene | 5.2 |
| Propane | 14.8 |
| Propylene | 15.8 |
| i-Butane | 1.7 |
| n-Butane | 2.1 |
| Butenes | 24.8 |
| $C_5$ Paraffins & Olefins (P&O) | 1.2 |
| $C_6$ P&O | 14.7 |
| $C_7$ P&O | 7.7 |
| $C_8$ P&O | 5.9 |
| $C_9$ P&O | 0.7 |
| Benzene | 0.1 |
| Toluene | 0.3 |
| Xylenes | 0.6 |
| $C_9$ Aromatics | 0.3 |

The product from this conversion reaction demonstrates utility for manufacture of a wide range of useful products. For instance, 4.1 wt. % $C_1$-$C_2$ paraffins, useful as fuel gas, were formed. The product included 18.6 wt. % $C_3$-$C_4$ paraffins, useful as LPG, and 45.8 wt. % $C_2$-$C_4$ olefins, useful as petrochemicals. The product also contained 31.5 wt. % $C_5$ + gasoline components.

## Claims

1. A synthetic porous crystalline material having an X-ray diffraction pattern exhibiting the following values:

| Interplanar d-Spacing (A) | | Relative Intensity, I/Io |
|---|---|---|
| 13.70 | ± 0.20 | W |
| 11.53 | ± 0.20 | W-VS |
| 10.38 | ± 0.20 | W |
| 7.82 | ± 0.14 | W-VS |
| 6.93-6.79 | ± 0.14 | W-VS |
| 6.19 | ± 0.14 | W-VS |
| 5.94 | ± 0.12 | W-M |
| 5.77 | ± 0.12 | VS |
| 5.22 | ± 0.12 | W |
| 5.18 | ± 0.10 | VS |
| 4.86 | ± 0.09 | M-S |
| 4.72 | ± 0.08 | S |
| 4.57 | ± 0.08 | W |
| 4.51 | ± 0.08 | S |
| 4.43 | ± 0.08 | W |
| 4.19 | ± 0.08 | W |
| 4.15 | ± 0.08 | M |
| 4.00 | ± 0.07 | W |
| 3.97 | ± 0.07 | W |
| 3.89 | ± 0.07 | W |
| 3.84 | ± 0.07 | M |
| 3.81 | ± 0.07 | W-M |
| 3.59 | ± 0.06 | W |
| 3.46 | ± 0.06 | W-M |
| 3.41 | ± 0.06 | S-VS |
| 3.36 | ± 0.06 | S-VS |
| 3.32 | ± 0.06 | M-S |
| 3.29 | ± 0.05 | W |
| 3.17 | ± 0.05 | W-M |
| 3.07 | ± 0.05 | W-M |
| 3.05 | ± 0.05 | W-M |
| 3.01 | ± 0.05 | W-M |
| 2.88 | ± 0.05 | W |
| 2.85 | ± 0.05 | W |
| 2.75 | ± 0.05 | W |
| 2.67 | ± 0.04 | W |
| 2.60 | ± 0.04 | W |

wherein the symbols indicating relative intensity have the following values:

W (weak) = 0–20
M (medium) = 20–40
S (strong) = 40–60
VS (very strong) = 60–100.

2. The crystalline material of Claim 1 having a composition in the as-synthesized form, on an anhydrous basis and in terms of moles of oxides per 100 moles of silica, expressed by the formula:

$(0.1–2.0)R_2O:(0.02–1.0)M_{2/n}O:(0.1–2)Al_2O_3:(100)SiO_2$

wherein M is an alkali or alkaline earth metal, n is the valence of M, and R is the methyltropinium cation.

3. The crystalline material resulting from thermal treatment of the crystalline material of Claim 2.

4. A method for preparing the synthetic crystalline material of Claim 1, said method comprising preparing a reaction mixture containing sources of an alkali metal or alkaline earth metal oxide, an oxide of silicon, an oxide of aluminum, water and methyltropinium salt directing agent of the formula (I), wherein X is an anion, and maintaining said reaction mixture under crystallization conditions until crystals of said material are formed.

5. The method of Claim 4 wherein said reaction mixture has a composition in terms of mole rations within the following ranges:

$$SiO_2/Al_2O_3 = 50 \text{ to } 1000$$
$$H_2O/SiO_2 = 5 \text{ to } 200$$
$$OH^-/SiO_2 = 0 \text{ to } 2.0$$
$$M/SiO_2 = 0.01 \text{ to } 3.0$$
$$R/SiO_2 = 0.01 \text{ to } 2.0$$

wherein M is alkali metal or alkaline earth metal and R is methyltropinium.

6. The method of Claim 4 wherein said reaction mixture has a composition in terms of mole ratios within the following ranges:

$$SiO_2/Al_2O_3 = 70 \text{ to } 500$$
$$H_2O/SiO_2 = 10 \text{ to } 100$$
$$OH^-/SiO_2 = 0.10 \text{ to } 1.0$$
$$M/SiO_2 = 0.10 \text{ to } 1.0$$
$$R/SiO_2 = 0.10 \text{ to } 0.50$$

wherein M is alkali metal or alkaline earth metal and R is methyltropinium.

7. The method of any one of Claims 4 to 6, wherein said reaction mixture further comprises seed crystals of said crystalline material.

8. The method of any one of Claims 4 to 7 including the further step of heating said crystalline material at least partly to remove the directing agent.

9. A process for converting an organic feedstock comprising contacting said feedstock under conversion conditions with a catalyst comprising a synthetic porous crystalline material as claimed in Claim 1 or Claim 3 or as produced by a method as claimed in Claim 8.

10. The process of Claim 9 wherein the feedstock comprises aromatic compounds selected from the group consisting of benzene, monocyclic alkyl-substituted benzene of from 7 to 12 carbon atoms and mixtures thereof, alkyl being methyl, ethyl or a combination thereof, and the conversion being alkylation, transalkylation, disproportionation or isomerization.

11. The process of Claim 9 wherein the feedstock comprises an organic oxygenate selected from an alcohol, carbonyl, ether or muxture thereof and the conversion product comprises hydrocarbon compounds.

**Patentansprüche**

1. Synthetisches poröses kristallines Material mit einem Röntgenbeugungsdiagramm, das die folgenden Werte aufweist:

| d-Netzebenenabstand (A) | Relative Intensität, I/Io |
|---|---|
| 13,70 ± 0,20 | W |
| 11,53 ± 0,20 | W-VS |
| 10,38 ± 0,20 | W |
| 7,82 ± 0,14 | W-VS |
| 6,93-6,79 ± 0,14 | W-VS |
| 6,19 ± 0,14 | W-VS |
| 5,94 ± 0,12 | W-M |
| 5,77 ± 0,12 | VS |
| 5,22 ± 0,12 | W |
| 5,18 ± 0,10 | VS |
| 4,86 ± 0,09 | M-S |
| 4,72 ± 0,08 | S |
| 4,57 ± 0,08 | W |
| 4,51 ± 0,08 | S |
| 4,43 ± 0,08 | W |
| 4,19 ± 0,08 | W |
| 4,15 ± 0,08 | M |
| 4,00 ± 0,07 | W |
| 3,97 ± 0,07 | W |
| 3,89 ± 0,07 | W |
| 3,84 ± 0,07 | M |
| 3,81 ± 0,07 | W-M |
| 3,59 ± 0,06 | W |
| 3,46 ± 0,06 | W-M |
| 3,41 ± 0,06 | S-VS |
| 3,36 ± 0,06 | S-VS |
| 3,32 ± 0,06 | M-S |
| 3,29 ± 0,05 | W |
| 3,17 ± 0,05 | W-M |
| 3,07 ± 0,05 | W-M |
| 3,05 ± 0,05 | W-M |
| 3,01 ± 0,05 | W-M |
| 2,88 ± 0,05 | W |
| 2,85 ± 0,05 | W |
| 2,75 ± 0,05 | W |
| 2,67 ± 0,04 | W |
| 2,60 ± 0,04 | W |

worin die die relative Intensität kennzeichnenden Symbole die folgende Werte aufweisen:

W (schwach) = 0–20
M (mittel) = 20–40
S (stark) = 40–60
VS (sehr stark) = 60–100.

2. Kristallines Material nach Anspruch 1 mit einer Zusammensetzung in der so synthetisierten Form auf wasserfreier Basis und auf die Mole der Oxide pro 100 Mole Siliciumdioxid bezogen, die durch die Formel ausgedrückt wird:

$(0.1–2,0)R_2O:(0,02–1,0)M_{2/n}O:(0,1–2)Al_2O_3:(100)SiO_2$,

worin M ein Alkali- oder Erdalkalimetall ist, n die Wertigkeit von M ist, und R das Methyltropiniumkation ist.

3. Kristallines Material, das aus der thermischen Behandlung des kristallinen Materials vom Anspruch 2 resultiert.

4. Verfahren zur Herstellung des synthetischen kristallinen Materials nach Anspruch 1, wobei dieses Verfahren die Herstellung einer Reaktionsmischung, die Quellen eines Alkalimetall- oder Erdalkalimetalloxids, Siliciumoxid, Aluminiumoxid, Wasser und ein Leitmittel in Form eines Methyltropiniumsalzes der Formel (I) enthält, worin X ein Anion ist, und das Halten dieser Reaktionsmischung bei Kristallisationsbedingungen umfaßt, bis Kristalle dieses Materials gebildet sind.

5. Verfahren nach Anspruch 4, worin die Reaktionsmischung eine auf die Molverhältnisse bezogene Zusammensetzung innerhalb der folgenden Bereiche aufweist

$SiO_2/Al_2O_3$ = 50 bis 100
$H_2O/SiO_2$ = 5 bis 200
$OH^-/SiO_2$ = 0 bis 0,2
$M/SiO_2$ = 0,01 bis 3,0
$R/SiO_2$ = 0,01 bis 2,0

worin M ein Alkalimetall oder Erdalkalimetall ist, und R Methyltropinium ist.

6. Verfahren nach Anspruch 4, worin die Reaktionsmischung eine auf die Molverhältnisse bezogene Zusammensetzung innerhalb der folgenden Bereiche aufweist:

$SiO_2/Al_2O_3$ = 70 bis 500
$H_2O/SiO_2$ = 10 bis 100
$OH^-/SiO_2$ = 0,10 bis 1,0
$M/SiO_2$ = 0,10 bis 1,0
$R/SiO_2$ = 0,10 bis 0,50

worin M ein Alkalimetall oder Erdalkalimetall ist, und R Methyltropinium ist.

7. Verfahren nach einem der Ansprüche 4 bis 6, worin die Reaktionsmischung außerdem Kristallkeime des kristallinen Materials enthält.

8. Verfahren nach einem der Ansprüche 4 bis 7, das den weiteren Schritt der zumindest teilweisen Erwärmung des kristallinen Materials umfaßt, um das Leitmittel zu entfernen.

9. Verfahren zur Umwandlung eines organischen Ausgangsmaterials, das den Kontakt des Ausgangsmaterials bei Umwandlungsbedingungen mit einem Katalysator umfaßt, der ein synthetisches poröses kristallines Material nach Anspruch 1 oder Anspruch 3 oder der nach dem Verfahren nach Anspruch 8 hergestellt wurde.

10. Verfahren nach Anspruch 9, worin das Ausgangsmaterial aromatiache Verbindungen umfaßt, die aus der Gruppe ausgewählt sind, die aus Benzol, monozyklischem mit Alkyl substituiertem Benzol mit von 7 bis 12 Kohlenstoffatomen und Mischungen davon besteht, wobei das Alkyl Methyl, Ethyl oder eine Kombination davon ist, und die Umwandlung die Alkylierung, Transalkylierung, Disproportionierung oder Isomerisation ist.

11. Verfahren nach Anspruch 9, worin das Ausgangsmaterial eine organische sauerstoffhaltige Verbindung umfaßt, die aus Alkohol, Carbonyl, Ether oder einer Mischung davon ausgewählt ist, und das Umwandlungsprodukt Kohlenwasserstoffverbindungen umfaßt.

**Revendications**

1. Matériau cristallin poreux de synthèse présentant une image de diffraction des rayons X correspondant aux valeurs suivantes:

| Espacement interplanaire d (Å) | Intensité relative, I/Io |
|---|---|
| 13,70 ± 0,20 | W |
| 11,53 ± 0,20 | W-VS |
| 10,38 ± 0,20 | W |
| 7,82 ± 0,14 | W-VS |
| 6,93-6,79 ± 0,14 | W-VS |
| 6,19 ± 0,14 | W-VS |
| 5,94 ± 0,12 | W-M |
| 5,77 ± 0,12 | VS |
| 5,22 ± 0,12 | W |
| 5,18 ± 0,10 | VS |
| 4,86 ± 0,09 | M-S |
| 4,72 ± 0,08 | S |
| 4,57 ± 0,08 | W |
| 4,51 ± 0,08 | S |
| 4,43 ± 0,08 | W |
| 4,19 ± 0,08 | W |
| 4,15 ± 0,08 | M |
| 4,00 ± 0,07 | W |
| 3,97 ± 0,07 | W |
| 3,89 ± 0,07 | W |
| 3,84 ± 0,07 | M |
| 3,81 ± 0,07 | W-M |
| 3,59 ± 0,06 | W |
| 3,46 ± 0,06 | W-M |
| 3,41 ± 0,06 | S-VS |
| 3,36 ± 0,06 | S-VS |
| 3,32 ± 0,06 | M-S |
| 3,29 ± 0,05 | W |
| 3,17 ± 0,05 | W-M |
| 3,07 ± 0,05 | W-M |
| 3,05 ± 0,05 | W-M |
| 3,01 ± 0,05 | W-M |
| 2,88 ± 0,05 | W |
| 2,85 ± 0,05 | W |
| 2,75 ± 0,05 | W |
| 2,67 ± 0,04 | W |
| 2,60 ± 0,04 | W |

dans lesquelles les symboles indiquant l'intensité relative ont les valeurs suivantes:

W (faible)   = 0–20
M (moyen)   = 20–40
S (intense)   = 40–60
VS (très intense)  = 60–100

2. Matériau cristallin selon la revendication 1, ayant une composition, sous sa forme telle que synthétisée, et sur une base anhydre et exprimée en moles d'oxydes pour 100 moles de silice, exprimée par la formule suivante:

$(0,1–2,0)R_2O/(0,02–1,0)M_{2/n}O(0,1–2)Al_2O_3/(100)SiO_2$

dans laquelle:
M est un métal alcalin ou alcalino-terreux;
n est la valence de M; et
R est le cation méthyltropinium.
3. Matériau cristallin résultant du traitement thermique du matériau cristallin selon la revendication 2.
4. Procédé pour la préparation du matériau cristallin de synthèse de l'exemple 1, ledit procédé consistant à préparer un mélange réactionnel contenant des sources d'un oxyde d'un métal alcalin ou alcalinoterreux, un oxyde de silicium, un oxyde d'aluminium, de l'eau et un agent d'alignement du type sel de méthyltropinium de formule (I), dans laquelle X est un anion, et à maintenir ledit mélange réactionnel dans les conditions de cristallisation jusqu'à formation de cristaux dudit matériau.
5. Procédé selon la revendication 4, dans lequel ledit mélange réactionnel a une composition, exprimée par les proportions molaires, correspondant aux intervalles suivantes:

$SiO_2/Al_2O_3$  = 50 à 1000
$H_2O/SiO_2$  = 5 à 200
$OH^-/SiO_2$  = 0 à 2,0
$M/SiO_2$  = 0,01 à 3,0
$R/SiO_2$  = 0,01 à 2,0

dans lequel:
M est un métal alcalin ou un métal alcalino-terreux; et
R est le méthyltropinium.
6. Procédé selon la revendication 4, dans lequel ledit mélange réactionnel a une composition, exprimée par les proportions molaires correspondant aux intervalles suivants:

$SiO_2/Al_2O_3$  = 70 à 500
$H_2O/SiO_2$  = 10 à 100
$OH^-/SiO_2$  = 0,10 à 1,0
$M/SiO_2$  = 0,10 à 1,0
$R/SiO_2$  = 0,10 à 0,50

dans lequel:
M est un métal alcalin ou un métal alcalino-terreux; et
R est le métyhltropinium.
7. Procédé selon l'une quelconque des revendications 4 à 6, dans lequel ledit mélange réactionnel comprend en outre des germes cristallins dudit matériau cristallin.
8. Procédé selon l'une quelconque des revendications 4 à 7, comprenant l'étape supplémentaire consistant à chauffer au moins partiellement ledit matériau cristallin, pour éliminer l'agent d'orientation.
9. Procédé pour la conversion d'une charge organique, qui consiste à mettre en contact ladite charge, dans les conditions de conversion, avec un catalyseur comprenant un matériau cristallin poreux de synthèse selon la revendication 1 ou 3, ou tel que produit par un procédé selon la revendication 8.
10. Procédé selon la revendication 9, dans lequel la charge comprend des composés aromatiques choisis dans l'ensemble comprenant le benzène, les benzènes monocycliques alkyles substitués ayant de 7 à 12 atomes de carbone et leurs mélanges, alkyle désignant les radicaux méthyle, éthyle ou une de leurs combinaisons, et la conversion étant une alkylation, une transalkylation, une dismutation ou une isomérisation.
11. Le procédé selon la revendication 9, dans lequel la charge comprend un oxygénate organique choisi parmi les composés alcools, carbonyle, éther ou leurs mélanges, le produit de conversion comprenant des composés hydrocarbonés.